# EUROPEAN PATENT APPLICATION

(11) **EP 2 127 602 A1**
(43) Date of publication of application: **02.12.2009**
(21) Application number: 09006999.8
(22) Date of filing: 26.05.2009
(51) Int. Cl.: A61B 8/12

(54) **Ultrasound diagnostic apparatus**

(30) Priority: 29.05.2008 JP 2008141280
(71) Applicant: Olympus Medical Systems Corporation, Tokyo 151-0072 (JP)
(72) Inventor: Kozai, Shigenori, Tokyo 151-0072 (JP)
(74) Representative: von Hellfeld, Axel

(57) **Abstract**

The ultrasound diagnostic apparatus 1 includes: an ultrasound probe 2 having an ultrasound transducer 9 made up of one or more oscillation elements 9A for transmitting and receiving ultrasound to and from a subject; and an ultrasound image observation apparatus including a connecting connector 8 to which the ultrasound probe 2 is detachably connected and a CPU 20 for detecting abnormality in the ultrasound probe 2 connected through the connecting connector 8.

## Description

### BACKGROUND OF THE INVENTION

### 1. Field of the invention

The present invention relates to an ultrasound diagnostic apparatus that includes an ultrasound probe and an ultrasound image observation apparatus to which the ultrasound probe is detachably attached.

### 2. Description of the Related Art

Ultrasound diagnostic apparatuses transmit ultrasound from an ultrasound transducer of an ultrasound probe connected to an ultrasound image observation apparatus and receive an echo signal reflected from a living tissue with the same ultrasound transducer to generate information in a living body as an ultrasonographic image and display the generated ultrasonographic image on a display section such as a monitor.

Ultrasound probes of ultrasound diagnostic apparatuses include an electronic scanning type probe for scanning inside a body cavity by electronically driving an ultrasonic transducer and a mechanical scanning type probe for scanning inside a body cavity by mechanically rotating an ultrasound transducer.

For example, an electronic scanning type ultrasound diagnostic apparatus disclosed in Japanese Patent Application Laid-Open Publication No. 6-47043 includes an ultrasound transducer made up of a plurality of oscillation elements and scans inside a body cavity by electronically switching, in sequence, the oscillation elements to be driven, to acquire an ultrasound image.

In addition, a mechanical scanning type ultrasound diagnostic apparatus disclosed in Japanese Patent Application Laid-Open Publication No. 2001-333906 scans inside a body cavity by driving an ultrasound transducer made up of one oscillation element while mechanically rotating the ultrasound transducer, to acquire an ultrasound image.

### SUMMARY OF THE INVENTION

An ultrasound diagnostic apparatus according to the present invention includes: an ultrasound probe including an ultrasound transducer, the ultrasound transducer being made up of one or more oscillation elements for transmitting and receiving ultrasound to and from a subject; and an ultrasound image observation apparatus including a connecting connector to which the ultrasound probe is detachably connected, and a control section for detecting abnormality in the ultrasound probe connected through the connecting connector.

### BRIEF DESCRIPTION OF THE DRAWINGS

FIG 1 is a block diagram showing a configuration of an ultrasound diagnostic apparatus according to an embodiment of the present invention.
FIG. 2 is a view showing a display example of an operation check screen of the ultrasound diagnostic apparatus according to the embodiment of the present invention.
FIG 3 is a flowchart showing a flow of abnormality detection processing by a control section of an ultrasound image observation apparatus of the ultrasound diagnostic apparatus according to the embodiment of the present invention.
FIG. 4 is a flowchart showing a subroutine of a check mode processing in the flowchart shown in FIG. 3.

### DETAILED DESCRIPTION OF THE PREFERRED EMBODIMENT

Hereinafter, an embodiment of the present invention will be described with reference to the drawings.

### (An Embodiment)

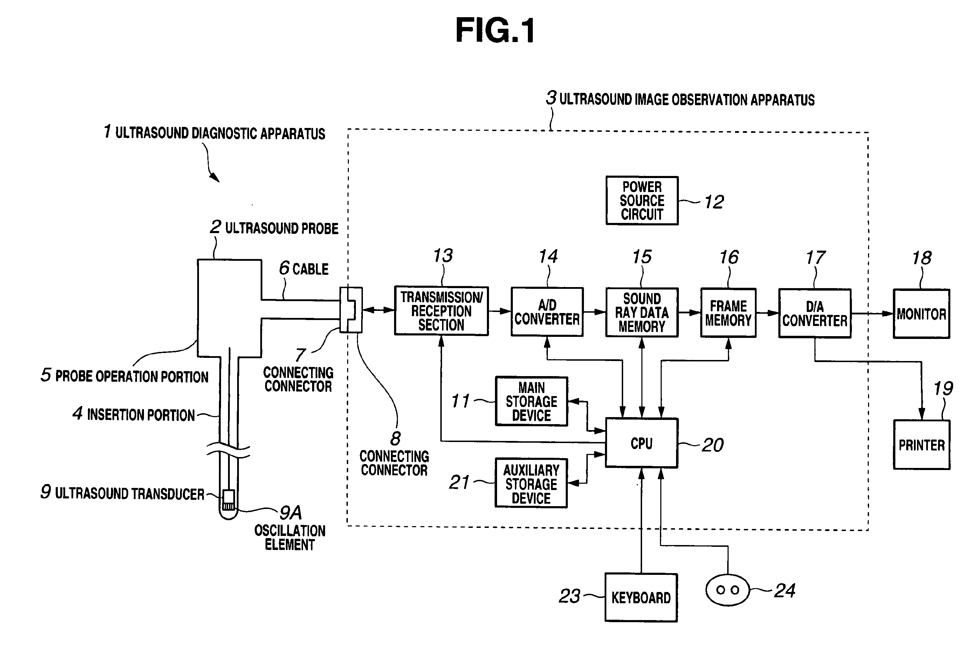

As shown in FIG. 1, an ultrasound diagnostic apparatus 1 according to the present embodiment includes an ultrasound probe 2 and an ultrasound image observation apparatus 3. The ultrasound probe 2 includes an ultrasound transducer 9 to be inserted into a body cavity of a subject, for transmitting and receiving ultrasound. The ultrasound transducer 9 performs a radial scan or a convex scan, for example. The ultrasound image observation apparatus 3 creates an image from an echo signal acquired by the ultrasound probe 2. That is, the ultrasound diagnostic apparatus 1 includes the ultrasound probe 2 that acquires an echo signal by transmitting and receiving ultrasound, and the ultrasound image observation apparatus 3 that displays the echo signal acquired by the ultrasound probe 2 as a two-dimensional image or a three-dimensional image.

The ultrasound probe 2 includes: an elongated insertion portion 4 to be inserted into a body cavity and the like through a channel of an endoscope; a probe operation portion 5 provided at a rear end of the insertion portion 4; and a cable 6 connected to the probe operation portion 5. At an end portion of the cable 6 is provided a connecting connector 7. The connecting connector 7 is detachably connected to a connecting connector 8 provided to the ultrasound image observation apparatus 3.

In a distal end portion of the insertion portion 4 of the ultrasound probe 2 according to the present embodiment is incorporated an ultrasound transducer 9 made up of one or more oscillation elements 9A, as an transducer array for performing a radial scan, for example.

The ultrasound image observation apparatus 3 includes: a transmission/reception section 13, an A/D converter 14; a sound ray data memory 15; a frame memory 16; a D/A converter 17; a microprocessor (hereinafter referred to as "CPU") 20; an auxiliary storage device 21; a main storage device 11; and a power source circuit 12.

The transmission/reception section 13 transmits and receives signals to and from the ultrasound probe 2 through the connecting connector 8. The A/D converter 14 converts an echo signal inputted from the transmission/reception section 13 into a digital signal. The sound ray data memory 15 is configured of a sound ray frame memory that stores a digital signal subjected to conversion in the A/D converter 14. Note that frame correlation processing may be performed on the echo signal to reduce its noise. The frame memory 16 stores digital ultrasound data for image display acquired by performing coordinate transformation for screen display, for example on a radial scan digital signal which is stored in the sound ray data memory 15. The D/A converter 17 converts the digital ultrasound data stored in the frame memory 16 into an analog image signal.

The CPU 20 as a control section controls driving of the transmission/reception section 13, the A/D converter 14, the sound ray data memory 15, and the frame memory 16, processes ultrasound data stored in the frame memory 16, and also controls the whole ultrasound diagnostic apparatus 1.

The auxiliary storage device 21 is a storage device that stores a software for instructing operation of the CPU 20 and various data. The main storage device 11 is configured of an RAM that temporarily stores the software and the like from the auxiliary storage device 21. The power source circuit 12 supplies driving power to each of the above-described circuits.

Furthermore, the ultrasound image observation apparatus 3 is connected with: a monitor 18, as display means, which displays an ultrasound image and additional information and the like based on the analog image signal outputted from the D/A converter 17; a printer device 19 which prints the ultrasound image and the like based on the analog image signal; a keyboard 23, as an ultrasound operation section, for an operator to operate the ultrasound diagnostic apparatus 1; and a foot switch 24.

The configuration and display state of the screen generated by the ultrasound image observation apparatus 3 and displayed on the monitor 18 are controlled by the CPU 20 as a control section. Note that the ultrasound image observation apparatus 3 may include an apparatus monitor section (not shown), as display means having the same working as that of the monitor 18, and a check screen and the like, which will be described later, may be displayed on the apparatus monitor section instead of the monitor 18, or on both of the apparatus monitor section and the monitor 18 at the same time.

Through the ultrasound operation section, the operator can input to the ultrasound image observation apparatus 3 an instruction of display range of an ultrasound image, a selecting instruction of the ultrasound probe 2 to be driven, a range switching instruction, and an instruction related to check mode processing of the ultrasound probe 2, and medical information such as patient information required for ultrasound inspection.

Note that a cursor and the like displayed on a display screen of the monitor 18 are operated using various keys, a trackball, or the like provided on the keyboard 23. Note that the operation on the display screen of the monitor 18 may be performed using a monitor and the like having a touch panel function. In this case, the monitor and the like having the touch panel function corresponds to the ultrasound operation section.

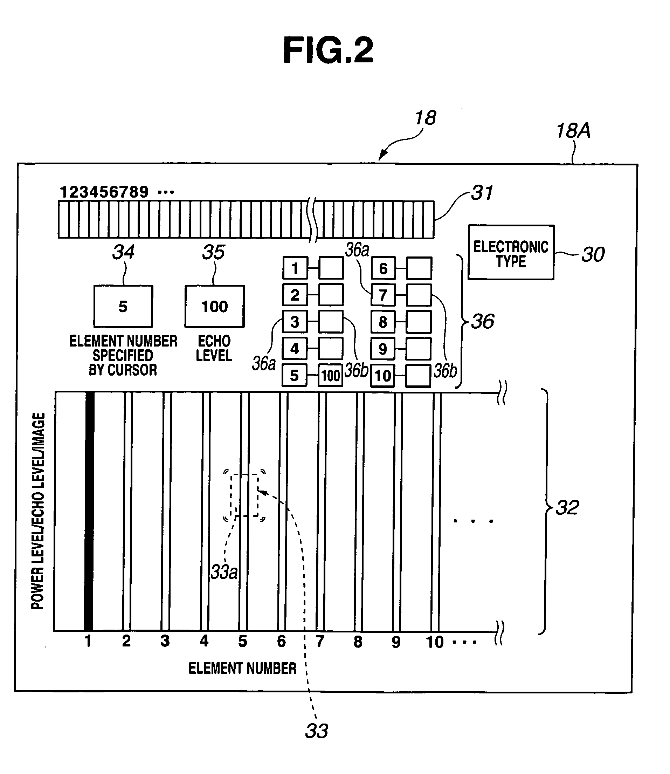

Furthermore, description will be made on the configuration of the ultrasound image observation apparatus 3 of the present embodiment and the display screen of the monitor 18 of the ultrasound image observation apparatus 3, with reference to FIGS. 1 and 2.

The ultrasound image observation apparatus 3 of the present embodiment transmits ultrasound with the ultrasound transducer 9 of the connected ultrasound probe 2, and generates an ultrasound image by signal-processing an echo signal acquired by receiving the ultrasound reflected by a living tissue of a subject. The ultrasound image observation apparatus 3 includes an abnormality detection function for detecting abnormality in the ultrasound probe 2 by checking the state of performance of the connected ultrasound probe 2.

The abnormality detection function of the ultrasound image observation apparatus 3 is implemented by execution of the software, that is, a program for check mode by the CPU 20 as a control section. The program for check mode is, for example, a program for checking the state of performance of the ultrasound probe 2 and detecting abnormality in the ultrasound probe 2 by checking whether or not each of the oscillation elements configuring the ultrasound transducer 9 normally transmits and receives ultrasound. The program for check mode is recorded in the auxiliary storage device 21 and executed by the CPU 20.

Note that the program for check mode contains, for example, transmission patterns and the like for causing each of the oscillation elements 9A of the ultrasound transducer 9 to generate a predetermined level of ultrasound, the transmission patterns and the like corresponding to each of the both types of the ultrasound probes, that is, electronic scanning type ultrasound probes and mechanical scanning type ultrasound probes.

In the present embodiment, the CPU 20 drives the transmission/reception section 13 in a predetermined transmission pattern by executing the program for check mode. The CPU 20 sequentially drives each of the oscillation elements 9A of the ultrasound transducer 9 one by one, to cause each of the oscillation elements 9A to transmit ultrasound pulses to a living tissue. The CPU 20 then acquires an echo signal for each of the oscillation elements 9A by the transmission/reception section 13. That is, the CPU 20 executes the check mode processing in which each of the oscillation elements 9A transmits ultrasound and acquires an echo signal, thereby acquiring operation check result of each of the oscillation elements 9A of the ultrasound transducer 9.

Then, the CPU 20 displays on the monitor 18 an operation check screen 18A including a check result display portion 32 on which the acquired operation check result of each of the oscillation elements 9A of the ultrasound transducer 9 is displayed.

FIG. 2 shows one example of the operation check screen 18A.

As shown in FIG. 2, the operation check screen 18A is configured of an ultrasound probe display portion 30, an ultrasound transducer display portion 31, the check result display portion 32, a cursor 33, an element number display portion 34, an echo level display portion 35, and a result display portion 36, for example.

On the ultrasound probe display portion 30 is displayed whether the connected ultrasound probe 2 is either the mechanical scanning type or the electronic scanning type. On the ultrasound transducer display portion 31 are displayed arrangement configuration and element numbers of the oscillation elements 9A of the ultrasound transducer 9 of the ultrasound probe 2. On the check result display portion 32 is displayed the result of check mode processing for each of the oscillation elements 9A. On the element number display portion 34 is displayed the element number of the oscillation element 9A specified by the operator using the cursor 33 on the check result display portion 32. On the echo level display portion 35 is displayed an echo level as intensity of the echo signal of the oscillation element 9A displayed on the element number display portion 34. In the result display portion 36, on an element number display portion 36a is displayed an element number of at least one oscillation element 9A which has ever been specified by the cursor 33, that is, the sound ray data number of the oscillation element 9A, and on an echo level display portion 36b is displayed the echo level of the echo signal of the sound ray data number.

Note that the shape of the cursor 33 is not limited to the rectangular shape as shown in FIG. 2, and may be a linear shape, for example.

On the check result display portion 32, the horizontal axis displays the element numbers of the oscillation elements 9A, and the vertical axis displays at least one of the echo level , acoustic power level (hereinafter referred to as "power level") of the generated ultrasound, and an ultrasound image, of the oscillation elements 9A of the respective element numbers. Note that it is not requisite to display the element numbers on the horizontal axis of the check result display portion 32, and only one of the echo level, the power level, and the ultrasound image may be displayable on the vertical axis.

For example, as the region indicating the element number 1 in FIG. 2, when there is abnormality in the oscillation element 9A of a predetermined element number, the region indicating the element number on the check result display portion 32 is displayed in black.

Note that the abnormality in the oscillation element 9A includes, for example, disconnection of a connection line to the oscillation element 9A or deterioration of the oscillation element 9A itself.

In the ultrasound diagnostic apparatus 1, the check result display portion 32 is displayed on the operation check screen 18A, so that the operator can easily identify the abnormality in the ultrasound transmitted from the ultrasound transducer 9 of the connected ultrasound probe 2, that is, the element number of oscillation element 9A having abnormality.

Depending on how many of the oscillation elements 9A have abnormality and where the oscillation elements 9A having abnormality are located in the ultrasound transducer 9, the operator can determine whether to immediately repair the ultrasound probe 2 having abnormality or to continue to use the ultrasound probe 2 having abnormality until an alternative ultrasound probe is sent from a manufacturer and becomes available.

Note that, when the cursor 33 and an ultrasound image are displayed on the operation check screen 18A and the like in a superimposed manner, it is preferable that the CPU 20 perform control to transparently display or not to display at least a part of the cursor 33 so that the operator can easily recognize the ultrasound image.

In addition, in the present embodiment, the CPU 20 can also perform control to change at least either a gain setting or a contrast setting of the specified ultrasound image, based on the specification using the cursor on the operation check screen 18A by the ultrasound operation section. That is, by operating the cursor on the operation check screen 18A, the operator can change at least either the gain setting or the contrast setting of the ultrasound image.

When the ultrasound image observation apparatus 3 can be connected with both the electronic scanning type ultrasound probe and the mechanical scanning type ultrasound probe at the same time, and when both types of the ultrasound probes are connected to the ultrasound image observation apparatus 3 at the same time, the CPU 20 can perform control to drive only one of the ultrasound probes based on the operator's instruction through the ultrasound operation section.

Furthermore, upon acquiring the operation check result for each of the oscillation elements 9A, the CPU 20 can compare an echo level threshold as a reference for abnormality determination of the echo level, which is prestored in a memory such as the auxiliary storage device 21, with the echo level of the oscillation element 9A and display the comparison result on the check result display portion 32. In the ultrasound image observation apparatus 3, the CPU 20 displays the comparison result obtained by comparing the echo level of the oscillation element 9A with the echo level threshold, which enables the operator to rapidly recognize whether or not the ultrasound transducer 9 normally operates.

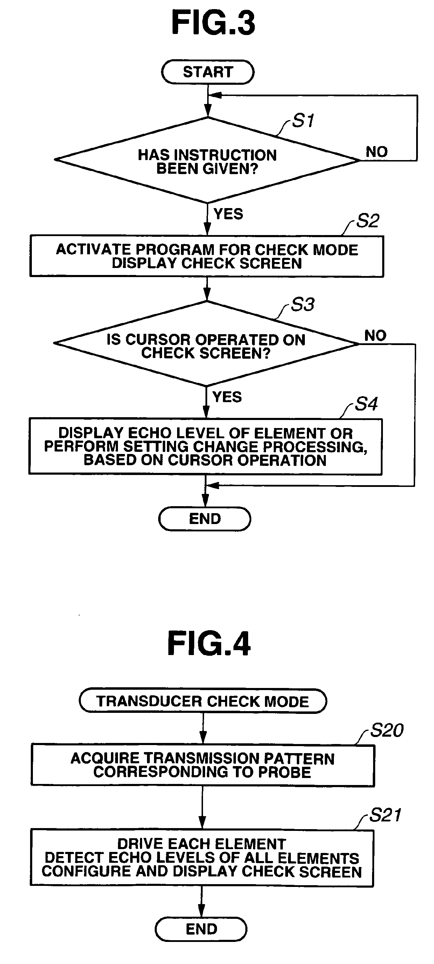

Next, description will be made on a flow of the processing by the ultrasound image observation apparatus 3 according to the present embodiment, with reference to FIGS. 3 and 4.

In step S1, the CPU 20 determines whether or not an instruction to start check mode processing has been given from the operator through the ultrasound operation section. When the instruction has been given (YES), the process moves on to step S2. When no instruction has been given (NO), the determination processing in step S1 is repeated until the instruction to start the check mode processing is given.

When the instruction to start the check mode processing is given, the CPU 20 reads out a program for check mode from the auxiliary storage device 21 and executes the program in the processing in step S2. The CPU 20 drives each of the oscillation elements 9A of the ultrasound transducer 9 and displays the acquired operation check result on the operation check screen 18A.

FIG. 4 shows a flow of processing of a subroutine program as a specific processing in step S2. When the processing in step S2 is executed, the CPU 20 executes processing according to the subroutine program starting from step S20.

In the processing in step S20, the CPU 20 reads out from the auxiliary storage device 21 a transmission pattern corresponding to the type of the connected ultrasound probe 2.

Then, in the processing in step S21, based on the transmission pattern acquired in the step S20, the CPU 20 drives the transmission/reception section 13 to cause each of the oscillation elements 9A to transmit ultrasound pulse and acquires an echo signal for each of the oscillation elements 9A by the transmission/reception section 13.

Then, the CPU 20 uses the acquired echo signal for each of the oscillation element 9A and configures the operation check screen 18A including the check result display portion 32 displaying the check mode processing result, and thereafter causes the monitor 18 to display the configured screen. After the processing according to the subroutine program, the CPU 20 performs processings in step S3 and steps subsequent thereto shown in FIG. 3.

In the processing in step S3, the CPU 20 determines whether or not an instruction is given from the operator using the cursor 33 displayed on the operation check screen 18A. When an instruction has been given, the CPU 20 moves on to the processing in step S4, and when no instruction is given, the CPU 20 terminates the program for check mode to perform preparatory operation for starting an inspection of a subject.

In the processing in step S4, when the operator operates the cursor 33 on the check result display portion 32 of the operation check screen 18A to place the cursor 33 on a certain element number, for example, the CPU 20 displays on the element number display portion 34 the element number on which the cursor 33 is placed, and also displays on the echo level display portion 35 the echo level of the oscillation element 9A having the element number.

Furthermore, when the operator operates the cursor 33 to give an instruction to change setting, the CPU 20 performs control to change at least either the gain setting or the contrast setting of the specified ultrasound image, for example.

The operation check screen 18A is displayed on the monitor 18 by the control performed by the CPU 20, which enables the operator to identify the state of the ultrasound probe 2, more specifically, the abnormality in the ultrasound probe 2.

The CPU 20 causes the operator to recognize the identified abnormality in the ultrasound probe 2, and thereafter terminates the program for check mode to perform preparatory operation for inspection of the subject.

According to the ultrasound image observation apparatus 3 of the present embodiment, it is not necessary for the operator to immediately send the whole ultrasound image observation apparatus 3 to the manufacturer for repairs even when some abnormality occurs. That is, when it is determined that abnormality has occurred in the ultrasound probe 2, the operator has only to repair the ultrasound probe 2. If the operator has a spare ultrasound probe 2, the operator can continue to use the ultrasound image observation apparatus 3.

Furthermore, the manufacturer who does repairs can also identify the cause of abnormality in the connected ultrasound probe 2 with a simple configuration and reduced cost, without using a dedicated inspection instrument or a dedicated program.

Note that description was made above, as the check mode, on the transducer check mode in which the CPU 20 causes each of the oscillation elements 9A of the ultrasound transducer 9 to generate ultrasound and displays on the check result display portion 32 the echo level of the echo signal of each of the oscillation elements 9A. However, the check mode is not limited to the transducer check mode, and for example, may be a power check mode in which the CPU 20 measures the power level of the ultrasound transmitted from each of the oscillation elements 9A of the ultrasound transducer 9 and displays the measured power level on the check result display portion 32.

In the power check mode, the CPU 20, for example, executes a program for measuring the power level for each of the oscillation elements 9A, that is, for each sound ray data. When the connected ultrasound probe 2 is the mechanical scanning type ultrasound probe 2, the CPU 20 stops the rotation of the ultrasound transducer 9 and thereafter drives the ultrasound transducer 9 to cause it generate ultrasound, and measures the power level at this time.

Note that the power level is measured using a hydrophone, not shown, with what is called hydrophone method. As a specific measuring method of power level with the hydrophone method, the method disclosed in the following is used, for example. (International Electrotechnical Commission: IEC 60601-2-37:2001, Medical electrical equipment-Part 2-37: Particular requirements for the basic safety and essential performance of ultrasonic medical diagnostic and monitoring equipment.)

On the other hand, when the connected ultrasound probe 2 is the electronic scanning type ultrasound probe 2, the CPU 20 drives only the oscillation element 9A which generates ultrasound in an arbitrarily predetermined one direction of the ultrasound transducer 9 and measures the power level for each sound ray.

Then, the CPU 20 compares the power level threshold, as a reference for the determination of abnormality in the power level, which is prestored in a memory such as the auxiliary storage device 21, with the measured power level for each sound ray, and configures the comparison result as the check result display portion 32 to cause the monitor 18 to display the operation check screen 18A.

According to this configuration, the power check mode can also provide the same effects as those in the transducer check mode in which the echo level is measured.

Note that when executing the power check mode, the CPU 20 can continuously drive only the oscillation elements related to one sound ray in a predetermined direction.

In addition, the CPU 20 can perform control to specify or change the oscillation element to be checked by operating the cursor 33 on the screen of the monitor 18 or the operation check screen 18A, for example.

Furthermore, when executing the power check mode, the CPU 20 can display a transmission parameter, that is, a transmission mode, frequency, focus position and the like of the ultrasound transmitted by the ultrasound transducer 9 on the screen of the monitor 18, for example, on the operation check screen 18A using the cursor 33, or perform control to change the transmission parameter by operating the cursor 33. Note that when the power level is measured using the hydrophone, it is possible to cause the ultrasound image observation apparatus 3 to output a trigger signal, for example, a frame sync required for the measurement.

In the above embodiment, description was made above on the transducer check mode and the power check mode as the abnormality detection function for detecting abnormality in the ultrasound probe 2, assuming the case where the processings in these modes are started by the processing start instruction from the operator. However, these modes may be executed, for example, as what is called self-check function which is performed when the power source of the ultrasound image observation apparatus 3 is turned on.

In addition, in the abnormality detection function, abnormality is identified by checking the operation of the connected ultrasound probe 2. By using the operation check result, it is also possible to discriminatingly detect the abnormality in the ultrasound probe 2 and the abnormality in the ultrasound image observation apparatus 3.

Note that description was made above by taking the ultrasound probe to be inserted into the channel of the endoscope as an example. However, it is apparent that the same effects can be obtained even if an ultrasound scope which can be inserted alone into a body cavity, that is, an ultrasound endoscope is used. In the present invention, the ultrasound probe includes an ultrasound probe.

In addition, the ultrasound transducer 9 is not limited to a radial scanning type, and may be a convex scanning type and the like.

## Claims

1. An ultrasound diagnostic apparatus comprising:
an ultrasound probe including an ultrasound transducer, the ultrasound transducer being made up of one or more oscillation elements for transmitting and receiving ultrasound to and from a subject; and
an ultrasound image observation apparatus including a connecting connector to which the ultrasound probe is detachably connected, and a control section for detecting abnormality in the ultrasound probe connected through the connecting connector.

2. The ultrasound diagnostic apparatus according to claim 1, wherein
the control section detects abnormality in each of the one or more oscillation elements by causing each of the one or more oscillation elements to transmit and receive the ultrasound and checking operation of each of the one or more oscillation elements.

3. The ultrasound diagnostic apparatus according to claim 2, further comprising display means, wherein the control section displays on the display means an operation check screen as an operation check result of each of the one or more oscillation elements.

4. The ultrasound diagnostic apparatus according to claim 3, wherein
the control section drives each of the one or more oscillation elements to transmit ultrasound, signal-processes an echo signal as a reception signal received by each of the one or more oscillation elements, and displays on the operation check screen an ultrasound image acquired using each of the one or more oscillation elements.

5. The ultrasound diagnostic apparatus according to claim 4, further comprising an ultrasound operation section connected to the ultrasound image observation apparatus, wherein
the control section displays a sound ray data number and an echo level of the echo signal of the ultrasound image that is specified by a cursor displayed on the operation check screen based on operation of the ultrasound operation section.

6. The ultrasound diagnostic apparatus according to claim 5, wherein the control section stores the sound ray data number and the echo level that have ever been specified by the cursor and displays on the operation check screen the sound ray data number and the echo level.

7. The ultrasound diagnostic apparatus according to claim 5, wherein, when the cursor and the ultrasound image are displayed in a superimposed manner, the control section transparently display or does not display at least a part of the cursor.

8. The ultrasound diagnostic apparatus according to claim 4, wherein the control section performs, based on operation of the ultrasound operation section, control to change at least either a gain setting or a contrast setting of the ultrasound image on the operation check screen.

9. The ultrasound diagnostic apparatus according to claim 1, wherein the control section includes an auxiliary storage device for storing a software for detecting abnormality in the ultrasound probe.
